# EUROPEAN PATENT APPLICATION

(11) **EP 3 427 773 A1**
(43) Date of publication of application: **16.01.2019**
(21) Application number: 18183014.2
(22) Date of filing: 11.07.2018
(51) Int. Cl.: A61M 1/16

(54) **DIALYSATE POTASSIUM CONTROL DURING A DIALYSIS SESSION**

(30) Priority: 11.07.2017 US 201715647168
(71) Applicant: Medtronic, Inc., Minneapolis, MN 55432 (US)
(72) Inventor: BURNES, John E., Coon Rapids, MN Minnesota 55448 (US); POINDEXTER, Rebecca L, Minneapolis, MN Minnesota 55413 (US); GERBER, Martin T, Maple Grove, MN Minnesota 55369 (US)
(74) Representative: Maschio, Antonio

(57) **Abstract**

The present invention relates to devices, systems, and methods for controlling the concentration of potassium in dialysate in a closed loop potassium control system. The devices, systems, and methods can be compatible with any dialysis system including sorbent-based dialysis systems, single pass dialysis systems, or other multi-pass dialysis systems. The systems can use closed loop potassium control over potassium concentration in the dialysate to reduce the probability of patient arrhythmias. The potassium concentration can be controlled and personalized to a patient using certain predetermined patient parameters. Related systems, algorithms, and control systems are contemplated for optimizing the potassium concentration in the dialysate.

## Description

### CROSS-REFERENCE TO RELATED APPLICATIONS

This application is a continuation in part of U.S. Patent Application No. 13/424,525 filed 03-20-2012, which claims benefit of and priority to U.S. Provisional Application No. 61/480,528 filed April 29, 2011, now expired, which claims benefit of and priority to U.S. Provisional Application No. 61/480,530 filed April 29, 2011, now expired, which claims benefit of and priority to U.S. Provisional Application No. 61/480,532 filed April 29, 2011, now expired, which claims benefit of and priority to U.S. Provisional Application No. 61/480,544 filed April 29, 2011, now expired, which claims benefit of and priority to U.S. Provisional Application No. 61/480,539 filed April 29, 2011, now expired, which claims benefit of and priority to U.S. Provisional Application No. 61/480,541 filed April 29, 2011, now expired, which claims benefit of and priority to U.S. Provisional Application No. 61/480,535 filed April 29, 2011, now expired, and the disclosures of each of the above-identified applications are hereby incorporated by reference in their entirety.

### FIELD OF INVENTION

The present invention relates to devices, systems, and methods for controlling the concentration of potassium in dialysate during a dialysis session by monitoring potassium and indicators of potassium and dynamically adjusting the potassium concentration of a dialysate based on the monitored potassium or indicators of potassium. The adjustments to the potassium concentration in the dialysate can be performed during a dialysis session in real-time and/or on-demand. The devices, systems, and methods can be compatible with a sorbent-based dialysis therapy or single-pass dialysis systems as well as infusate sources and related systems and methods for adjusting the potassium dialysate concentration. Moreover, the potassium dialysate concentration can be controlled and personalized to a patient using predetermined patient parameters obtained from the sensors of the present invention. Related infusate delivery, dialysis, algorithms, sensing, and control systems are contemplated for optimizing the potassium concentration in the dialysate.

### BACKGROUND

Removal of potassium from a patient can be a critical function in for dialysis therapy such as hemodialysis, hemodiafiltration, and peritoneal dialysis. The amount and rate of potassium removal from the patient can depend on the potassium concentration of the dialysate. Even modest potassium changes in patients having cardiovascular or renal disease can increase the risk of hospitalization and death (Noninvasive potassium determination using a mathematically processed ECG: Proof of concept for a novel "blood-less, blood test," Dillon et al., J. Electrocadiology 48 (2015) 12-18). The sudden death rate triples for patients undergoing hemodialysis three times weekly in a 12 hour period prior to scheduled dialysis following a two-day, weekend hiatus (Characteristics of Sudden Death in Hemodialysis Patients, Bleyer et al., Kidney Int. 2006:69:2268-73). Removing potassium at too high a rate or not removing sufficient potassium can result in an increased likelihood of patient arrhythmia (Pathogenesis, diagnosis and management of hyperkalemia, Pediatric Neph. 2011 March 26(3): 377-384). As such, inappropriate dialysate potassium concentrations can negatively impact the rate of change or a concentration gradient in patient potassium levels, causing increased risk and/or incidence of arrhythmia.

However, known dialysis systems do not have mechanisms for profiling the potassium concentration in the dialysate to minimize patient arrhythmia due to a high or an insufficient rate of potassium removal. Known systems typically deliver a dialysate potassium concentration in a blind manner that does not depend upon the patient's instantaneous potassium level. As such, the known systems cannot provide a personalized dialysis prescription based on a individual's physiologic template at a known potassium value or detect changes based on the personalized template in a non-obtrusive, frequent manner during dialysis therapy. Moreover, the known systems cannot make adjustments to the potassium dialysate concentration tailored to the patient and to the particular dialysis therapy session. In other words, the known systems cannot dynamically adjust, control, monitor, or deliver an appropriate dialysate potassium concentration based on the instantaneous potassium level during a dialysis session. Yet, control over the rate of change in patient potassium levels can be a critical factor to reducing arrhythmia risk.

Hence, there is a need for systems and methods that can profile the potassium concentration of the dialysate in a system based on measurements of the potassium level of the patient. The need extends to sensing components, techniques, algorithms, and systems that can profile a patient's potassium levels and to providing data to dialysis systems capable of adjusting the dialysate in real-time or on-demand. The need extends to single pass systems or to systems that regenerate dialysate, and to sensors capable of monitoring the patient potassium level and communicating the patient potassium levels to a controller for adjusting the dialysate potassium concentration. The need includes automated systems for monitoring potassium, sensing potassium, and dynamically adjusting a potassium concentration in a dialysate during an ongoing dialysis session. There is further a need for systems that can set a dialysate potassium concentration based on the monitored patient potassium levels in real-time, taking into account the instantaneous potassium levels of the patient. The systems should be capable of delivering a potassium source and making adjustments to the potassium concentration source in real-time.

### SUMMARY OF THE INVENTION

The first aspect of the invention is drawn to a method.

Herein is described a method of adjusting the potassium concentration in a dialysate.

Said method comprises the steps of:
a) monitoring a potassium level in a fluid using a sensor;
b) adjusting potassium concentration in a dialysate based on the monitored potassium level; wherein the dialysate potassium concentration is adjusted to result in a potassium level in the fluid of within a specified range.

The dialysate is used to dialyze the fluid, thus modifying the potassium levels in the fluid. The potassium concentration in the dialysate can be adjusted to achieve desired potassium levels in the fluid.

The "specified range" of the potassium level is preferably a healthy dialysate potassium range for humans, adults or children. The specified range may be determined by measuring the dialysate potassium concentration of a cohort of healthy individuals.

The specified range preferably has an upper limit of 6.0 mmol/L or less, preferably 5.5 mmol/L or less, and most preferably 5.0 mmol/L or less. The specified range preferably has lower limit 2.5 mmol/L or more, preferably 3.0 mmol/L or more, and most preferably 3.5 mmol/L or more.

In preferable embodiments, the specified range of the potassium level is 3.5 - 5.0 mmol/L.

The method of the invention initiated on a closed loop potassium control system.

The step of monitoring a potassium level in the methods of the invention comprises monitoring a potassium gradient across a dialyzer, as defined herein.

The step of monitoring the potassium level of the dialysate may use any suitable sensor, such as an implantable sensor, external sensor, subcutaneous sensor, surface sensor, or a combination thereof. Such sensors may be used in vitro. Such sensors may be used to test the dialysate potassium levels in a sample or range of samples of dialysate from a patient or patients, wherein the dialysate may be in the form of a continuous flow.

Preferably a suitable sensor is an ECG or an EGM sensor.

The step of monitoring the potassium level of the dialysate is performed by a sensor on any one of a dialysis machine, external medical device, or point of care component.

The method of the invention may be adjusted in use. For example, the dialysate potassium concentration may be adjusted by adjusting a rate of addition of a potassium concentrate to the dialysate.

Preferably, during the methods of the invention, the potassium level of the dialysate is monitored and adjusted at specified time points or intervals. These time points may be 1 second, 2 second, 10 second, 30 seconds, 1 minute, 2 minutes, 3 minutes, 5 minutes, 10 minutes, 15 minutes, 30 minutes or 1 hour apart from example.

Preferably the method of the invention is performed by a dialysis system.

In one embodiment, during the methods of the invention the dialysate potassium concentration is adjusted to maintain a rate of change of the potassium level of the dialysate below a predetermined threshold. If a rate of change in the dialysate potassium level above a predetermined threshold is detected, the potassium concentration of the dialysate can be increased. The step of adjusting the dialysate potassium concentration comprises adjusting a rate of addition of a potassium concentrate from a potassium source into a dialysate flow path. Preferably an extracorporeal flow path.

In methods of the invention, the dialysate potassium concentration is adjusted to maintain a constant potassium gradient across a dialyzer, preferably wherein the dialysate potassium concentration is monitored with a potassium sensor in a dialysate flow path downstream of a dialyzer.

The method according to any preceding claim, further comprising adjusting a concentration of at least one other solute in a dialysate based on the monitored potassium level of the dialysate, preferably wherein the at least one other solute is either glucose or bicarbonate.

Also herein we describe a dialysis system comprising;
a dialysate flow path having a dialyzer;
a potassium sensor for determining potassium levels in a patient;
an extracorporeal flow path fluidly connected to the dialyzer;
a potassium source fluidly connected to the dialysate flow path;
a controller, the controller setting a rate of addition of potassium from the potassium source to the dialysate flow path; wherein the potassium sensor provides potassium level information to the controller to adjust potassium levels in the dialysate, and potassium levels in the dialysate are adjusted to achieve a potassium level in the patient within a specified range.

Preferably, the controller is programmed to set the rate of addition of potassium from the potassium source to the dialysate flow path to achieve a dialysate potassium concentration within a specified range.

The dialysis system preferably wherein the dialysate potassium concentration range is within the specified range described above, most preferably is 3.5 - 5.0 mmol/L.

Also herein are described methods which mayinclude initiating a dialysis session for a patient; monitoring a potassium level of the patient using a sensor;; and adjusting a dialysate potassium concentration during the dialysis session based on the monitored potassium level; wherein the dialysate potassium concentration is adjusted to result in a patient potassium level within. 3.5-5.0 mmol/L

In any embodiment, the method can use a closed loop potassium control system.

In any embodiment, the step of monitoring a potassium level can include monitoring a potassium level of the patient.

In any embodiment, the step of monitoring a potassium level can include monitoring the dialysate potassium concentration.

In any embodiment, the step of monitoring a potassium level can include monitoring a potassium gradient across a dialyzer.

In any embodiment, the method can include the step of monitoring a potassium level of the patient prior to initiating the dialysis session, and setting an initial dialysate potassium concentration based on the monitored potassium level of the patient.

In any embodiment, the step of monitoring the potassium level of the patient can use an implanted sensor.

In any embodiment, the sensor can be an ECG or an EGM sensor.

In any embodiment, the implanted sensor can be a potassium sensor.

In any embodiment, the step of monitoring a potassium level of the patient can be performed by a sensor on any one of a dialysis machine, external medical device, or point of care component.

In any embodiment, the step of monitoring a potassium level of the patient can be performed by any one of an implantable sensor, external sensor, subcutaneous sensor, surface sensor, and combinations thereof.

In any embodiment, adjusting the dialysate potassium concentration can include adjusting a rate of addition of a potassium concentrate to a dialysate.

In any embodiment, monitoring the potassium level of the patient and adjusting the dialysate potassium concentration can be repeated throughout the dialysis session.

In any embodiment, the method can be performed by a dialysis system.

In any embodiment, the potassium concentration of the dialysate can be adjusted to maintain a rate of change of the potassium level of the patient below a predetermined threshold.

In any embodiment, the dialysate potassium concentration can be adjusted to maintain a constant potassium gradient across the dialyzer.

In any embodiment, the step of adjusting the dialysate potassium concentration can include adding a potassium concentrate from a potassium source into a dialysate flow path.

In any embodiment, the method can include adjusting a concentration of at least one other solute in a dialysate based on the monitored potassium level of the patient.

In any embodiment, the at least one other solute can be either glucose or bicarbonate.

In any embodiment, the method can include monitoring a dialysate potassium concentration with a potassium sensor in a dialysate flow path downstream of a dialyzer.

In any embodiment, the sensor can be a potassium sensor in an extracorporeal flow path of a dialysis system.

In any embodiment, the method can include stopping the dialysis session in response to a potassium level of the patient below a predetermined threshold.

In any embodiment, the method can include alerting a user in response to a potassium level of the patient below a predetermined threshold.

In any embodiment, the step of adjusting the dialysate potassium concentration can include adjusting the dialysate potassium concentration according to a user selected response to the monitored potassium level of the patient.

In any embodiment, the method can include receiving a target potassium level of the patient; and the step of adjusting the dialysate potassium concentration can include adjusting the dialysate potassium concentration to result in the target potassium level of the patient.

In any embodiment, the step of adjusting the dialysate potassium concentration can include preventing a potassium level of the patient from decreasing below the target potassium level of the patient.

Any of the features disclosed as being part of the first aspect of the invention can be included in the first aspect of the invention, either alone or in combination.

The second aspect of the invention is drawn to a dialysis system. In any embodiment, the dialysis system can include a dialysate flow path having a dialyzer; an extracorporeal flow path fluidly connected to the dialyzer; a potassium source fluidly connected to the dialysate flow path; a controller, the controller setting a rate of addition of potassium from the potassium source to the dialysate flow path; wherein the controller is programmed to receive a potassium level of a patient and to set the rate of addition of potassium from the potassium source to the dialysate flow path based on the potassium level of the patient; wherein the controller is programmed to set a dialysate potassium concentration to result in a patient potassium level within 3.5 to 5.0 mmol/L.

In any embodiment, the controller can be programmed to receive the potassium level of the patient from an implanted sensor.

In any embodiment, the sensor can be an ECG or EGM sensor.

In any embodiment, the implanted sensor can be a potassium sensor.

In any embodiment, the controller can be programmed to receive the potassium level of the patient from a potassium sensor in the dialysate flow path downstream of the dialyzer.

In any embodiment, the system can include a sorbent based filtration system in the dialysate flow path.

In any embodiment, the sorbent based filtration system can include one or more sorbent cartridges.

In any embodiment, the system can be a single pass dialysis system.

In any embodiment, the controller can be programmed to set a rate of addition of potassium into the dialysate flow path based on a potassium level of the patient received during a dialysis session.

In any embodiment, the controller can be programmed to set the rate of addition of potassium into the dialysate flow path based on a rate of change in the potassium level of the patient.

In any embodiment, the controller can be programmed to maintain a constant potassium gradient across the dialyzer.

In any embodiment, the system can include a glucose source and/or a bicarbonate source fluidly connected to the dialysate flow path; wherein the controller is programmed to set a rate at which glucose and/or bicarbonate is added to the dialysate flow path based on the potassium level of the patient.

In any embodiment, the system can include a processor programmed to receive and store the potassium level of the patient.

In any embodiment, the processor can be further programmed to receive one or more additional parameters; the system can include an output; and the output can plot the potassium level of the patient and the one or more additional parameters.

In any embodiment, the additional parameters can be selected from a sodium level of the patient, a blood flow rate for a dialysis session, a dialysate flow rate for a dialysis session, a patient blood pressure, and a patient pulse rate.

The features disclosed as being part of the second aspect of the invention can be in the second aspect of the invention, either alone or in combination.

The third aspect of the invention is drawn to a method. In any embodiment, the method can include (a) setting a prescription for changing a potassium level of a patient from a baseline potassium level of the patient during a dialysis session; wherein the prescription for changing the potassium level of the patient includes either or both of (i) a defined potassium gradient across a dialyzer; and/or (ii) a defined rate of change in the potassium level of the patient; (b) initiating the dialysis session; and (c) adjusting a dialysate potassium concentration to maintain the defined potassium gradient across the dialyzer or the defined rate of change in the potassium level of the patient.

In any embodiment, the dialysis session can be initiated with a system having a sorbent based filtration system; wherein the sorbent based filtration system removes at least a portion of potassium in a dialysate.

In any embodiment, the baseline potassium level of the patient can be received from a sensor.

In any embodiment, the defined potassium gradient across the dialyzer can be set at or below a predetermined threshold.

In any embodiment, the defined rate of change in the potassium level of the patient can be set at or below a predetermined threshold.

The features disclosed as being part of the third aspect of the invention can be in the third aspect of the invention, either alone or in combination.

### BRIEF DESCRIPTION OF THE DRAWINGS

FIG. 1 shows a dialysis system for adjusting the dialysate potassium concentration during a dialysis session.
FIG. 2 is a flow chart of a method for adjusting the dialysate potassium concentration based on a potassium level of a patient.
FIG. 3 is a flow chart of a method for adjusting the dialysate potassium concentration based on a rate of change in the potassium level of a patient.
FIG. 4 is a flow chart for setting a prescription for changing the potassium level of a patient.
FIG. 5 is a schematic block diagram showing flow paths and closed loop control mechanisms for controlling flow of concentrate into fluid for use in a blood fluid removal process based on monitored pH or electrolytes.

### DETAILED DESCRIPTION OF THE INVENTION

Unless defined otherwise, all technical and scientific terms used have the same meaning as commonly understood by one of ordinary skill in the art.

The articles "a" and "an" are used to refer to one or to more than one (*i.e.,* to at least one) of the grammatical object of the article. For example, "an element" means one element or more than one element.

The terms "adjusting," or to "adjust" can refer to changing a variable or state of a system.

The terms "alerting" or to "alert" refer to informing a user of a potentially hazardous condition.

The term "baseline potassium level of the patient" refers to the potassium level of the patient prior to initiating a dialysis session.

The term "bicarbonate source" can refer to a source of bicarbonate ions in solid and/or solution form. The bicarbonate ions can be present as a bicarbonate salt of any type. The bicarbonate source can contain at least one fluid pathway and include components such as conduits, valves, filters or fluid connection ports, any of which are fluidly connectable to each other or to a fluid flow path. The bicarbonate source can either be formed as a stand-alone enclosure or a compartment integrally formed with an apparatus containing the bicarbonate source.

The term "bicarbonate" can refer to either bicarbonate anions or predecessors to bicarbonate anions, such as salts of bicarbonate, e.g. sodium bicarbonate. Predecessors to bicarbonate anions are any substances that can convert to bicarbonate anions based on conditions within a system or within a patient, such as acetate, lactate, and/or citrate.

"Blood flow rate" can refer to the rate at which blood is flowed through a system. For example, the blood flow rate can describe the rate of blood flow through a dialyzer during therapy.

The term "closed loop potassium control" refers to a system that uses one or more potassium sensors to control a level of potassium in a dialysate, thus controlling an amount of potassium removed from a patient or the patient's final potassium level. In contrast, an "open loop potassium control system" uses a set potassium level in the dialysate without regard to the patient potassium level.

The term "comprising" includes, but is not limited to, whatever follows the word "comprising." Use of the term indicates the listed elements are required or mandatory but that other elements are optional and may be present.

The term "consisting essentially of' includes whatever follows the term "consisting essentially of' and additional elements, structures, acts or features that do not affect the basic operation of the apparatus, structure or method described.

The term "consisting of' includes and is limited to whatever follows the phrase "consisting of." The phrase indicates the limited elements are required or mandatory and that no other elements may be present.

The term "constant potassium gradient" refers to a state in which a difference between potassium concentrations of fluids on opposing sides of a semi-permeable membrane is not changing by a significant amount.

A "controller" is a device which monitors and affects the operational conditions of a given system. The operational conditions are typically referred to as output variables of the system wherein the output variables can be affected by adjusting certain input variables.

The term "defined potassium gradient" refers to a pre-set difference in potassium concentrations in fluids separated by a semi-permeable membrane.

The term "defined rate of change" refers to a pre-set difference in magnitude of a parameter measured as a function of time.

The term "dialysate" describes a fluid into or out of which solutes from a fluid to be dialyzed diffuse through a membrane. A dialysate typically contains electrolytes close in concentration to the physiological concentration of electrolytes found in blood.

The term "dialysate flow path" can refer to a fluid pathway or passageway that conveys a fluid, such as dialysate and is configured to form at least part of a fluid circuit for peritoneal dialysis, hemodialysis, hemofiltration, hemodiafiltration or ultrafiltration.

"Dialysate flow rate" can refer to the rate at which dialysate is pumped through a dialysate flow path.

"Dialysate potassium concentration" can refer to the concentration of potassium ions in a dialysate.

The "dialysate regeneration module" can refer to a system for removing certain electrolytes and waste species, such as urea, from a dialysate after contact with a dialyzer. In certain instances, the components contained within the "dialysate regeneration modules" can decrease the concentration or conductivity of at least one ionic species, or release and/or absorb at least one solute from a dialysate.

"Dialysis" or "dialysis therapy" is a filtration, or a process of selective diffusion or convection through a membrane. Dialysis removes solutes of a specific range of molecular weights via diffusion through a membrane from a fluid to be dialyzed into a dialysate. During dialysis, a fluid to be dialyzed is passed over a filter membrane, while dialysate is passed over the other side of that membrane. Dissolved solutes are transported across the filter membrane by diffusion between the fluids or by convection through the membrane. The dialysate is used to remove solutes from the fluid to be dialyzed. The dialysate can also provide enrichment to the other fluid.

A "dialysis machine" is a system comprising a dialyzer, pumps, valves and fluid lines that is used to carry out a dialysis session.

A "dialysis session" is a period of time in which treatment of a patient by dialysis is ongoing.

The term "dialysis system" can refer to a set of components configured to carry out dialysis therapy of any type including peritoneal dialysis, hemodialysis, hemofiltration, hemodiafiltration or ultrafiltration.

The term "dialyzer" can refer to a cartridge or container with two flow paths separated by semi-permeable membranes. One flow path is for blood and one flow path is for dialysate. The membranes can be in hollow fibers, flat sheets, or spiral wound or other conventional forms known to those of skill in the art. Membranes can be selected from these materials: polysulfone, polyethersulfone, poly (methyl methacrylate), modified cellulose, or other materials known to those skilled in the art.

The term "downstream" refers to a position of a first component in a flow path relative to a second component wherein fluid will pass by the second component prior to the first component during normal operation. The first component can be said to be "downstream" of the second component, while the second component is "upstream" of the first component.

An "electrocardiogram" or "ECG sensor" refers to a sensor measuring a time varying waveform, produced by the electrical activity of the cardiac muscle and the associated electrical network within the myocardium. The term is often used interchangeably for the ECG tracing available from the surface of the subject, or from an implantable device.

An "electrogram" or "EGM sensor" measures electrical activity of the heart.

An "extracorporeal flow path" refers to a fluid pathway incorporating one or more components such as but not limited to conduits, valves, pumps, fluid connection ports or sensing devices configured therein such that the pathway conveys blood from a subject to an apparatus for hemodialysis, hemofiltration, hemodiafiltration or ultrafiltration and back to the subject.

An "external medical device" describes any device, component or module outside of a patient's body. An external medical device can refer, but is not limited to, any treatment device, monitoring device, or physiological data recording device.

An "external sensor" is a sensor located outside of the body of a patient.

A "fluid" is a gas phase or liquid phase substance optionally having a combination of gas and liquid phases.

The term "fluidly connectable," "fluidly connected," or "in fluid communication" refers to a structure, passageway, or ability to pass fluid, gas, or mixtures thereof from one point to another point. The two points can be within or between any one or more of compartments, modules, systems, and components.

"Glucose" refers to a naturally occurring carbohydrate having the chemical formula C₆H₁₂O₆.

The term "glucose source" can refer to a source of glucose in solid and/or solution form. The glucose source can contain at least one fluid pathway and include components such as conduits, valves, filters or fluid connection ports, any of which are fluidly connectable to each other or to a fluid flow path. The glucose source can either be formed as a stand-alone enclosure or a compartment integrally formed with an apparatus containing the glucose source.

The term "implanted" describes a device, component, or sensor intended to be totally or partially introduced, surgically or medically into a mammalian body, or by medical intervention that remains after the procedure.

An "implantable sensor" is a sensor located within the body of a patient.

The term 'initiate a dialysis session" or "initiating a dialysis session" means to begin treatment of a patient by dialysis.

An "initial dialysate potassium concentration" is a concentration of potassium ions in a dialysate at the beginning of a dialysis session.

The terms "maintaining" or to "maintain" refer to preventing a parameter or state of a system from changing by a significant amount.

The terms "monitoring" or to "monitor" mean to collect data regarding one or more parameters, preferably patient parameters. Monitoring can be in real time. Monitoring can be carried out *in vitro.* In preferable embodiments, monitoring involves collecting data, such as dialysate potassium concentration, in a sample or series of samples taken from a patient or patient, which may be in the form of a continuous flow of dialysate.

An "output" is a component or set of components capable of providing information to a user.

The term "parameter" of a subject represents a characteristic, feature, or measurable factor that helps define the subject. A parameter can have a nominal description to define the subject qualitatively, and can also be a quantitative value or a certain range of quantitative values, regarding a certain feature of the subject. For example, a parameter of a patient can describe any features related to the patient, including concentration of a certain component in the blood, such as "blood urea nitrogen," "total body water," which can be calculated anthropometrically using the Hume-Weyers equations depending on gender, height, and weight of the individual of interest. A parameter of a hemodialysis system can describe any features of any device or fluid in the system. In statistics, a parameter is often used interchangeably with the terms of "variable" and "factor." A variable denotes a mathematical object, representing value that can be specified and can be varied. Parameters can also be the names of variables in the function.

A "patient" or "subject" is a member of any animal species, preferably a mammalian species, optionally a human. The subject can be an apparently healthy individual, an individual suffering from a disease, or an individual being treated for a disease.

"Patient blood pressure" or "blood pressure" refers to the pressure of blood in the body of a patient and can refer to systolic pressure, diastolic pressure, or a combination thereof.

The term "patient pulse rate" refers to the number of heartbeats for a patient in a given time period.

The terms "plotting" or to "plot" refer to creating a graphical representation of one or more parameters.

A "point of care component" is any medical device, monitor, or sensor available at a health care facility.

The term "potassium concentrate" can refer to a fluid having a potassium concentration higher than a potassium concentration to be used in a resulting fluid obtained from the potassium concentrate.

The term "potassium gradient" refers to a difference in potassium concentrations in fluids separated by a semi-permeable membrane.

The term "potassium level" can refer to a concentration of potassium ions in either a fluid or a patient.

The term "potassium level of the patient" can refer to the concentration of potassium ions in a patient at any given point in time.

A "potassium sensor" is a component measuring the potassium concentration in a patient or system.

A "potassium source" can refer to a source of potassium ions in solid and/or solution form. The potassium ions can be present as a potassium salt of any type. The potassium source can contain at least one fluid pathway and include components such as conduits, valves, filters or fluid connection ports, any of which are fluidly connectable to each other or to a fluid flow path. The potassium source can either be formed as a stand-alone enclosure or a compartment integrally formed with an apparatus containing the potassium source

The term "predetermined threshold" can refer to a predetermined value for a parameter, wherein an action taken can depend on the pre-set threshold value of the parameter.

A "prescription for changing a potassium level of a patient" refers to a change in patient potassium level of a patient or a rate of change in potassium level of the patient set for a dialysis session.

The term "prevent" or "preventing" means to cause a specified outcome not to occur.

The term "processor" as used herein is a broad term and is to be given its ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. The terms can include ROM ("read-only memory") and/or RAM ("random-access memory") associated therewith.

The term "programmed," when used referring to a processor or computer, can refer to a series of instructions that cause a processor, software, hardware, or computer to perform certain steps.

The term "rate of addition" refers to the amount of a substance added to a flow path as a function of time.

The term "rate of change" refers to a difference in magnitude of a parameter measured as a function of time.

The term "receive" means to obtain data from a any source.

The term "removes at least a portion of potassium" refers to a filtration system in which a fluid having potassium ions passing through the filtration system will exit the filtration system with a lower concentration of potassium ions.

The terms "repeated" or to "repeat" refer to performing the same or similar acts multiple times.

The term "result" means to cause a specified outcome.

A "sensor" is a component capable of determining one or more states of one or more variables in a system.

"Setting," "to set," and the like, can refer to an act whether implemented by a processor, computer, or algorithm wherein a parameter or flow rate is controlled to result in a desired result.

A "single pass dialysis system" is a dialysis system in which spent dialysate is continuously discarded and new dialysate generated for treatment.

The term "sodium level of the patient" can refer to the concentration of sodium ions in a patient at any given point in time

A "solute" is a substance dissolved in, or intended to be dissolved in, a solvent.

The term "sorbent based filtration system" refers to a system that removes substances from a dialysate by adsorption. The sorbent based filtration system can include one or more sorbent cartridges or any other components with a sorbent material.

The terms "sorbent cartridge" and "sorbent container" refer to a cartridge containing one or more sorbent materials for removing specific solutes from solution, such as urea. The term "sorbent cartridge" does not require the contents in the cartridge be sorbent based, and the contents of the sorbent cartridge can be any contents that can remove waste products from a dialysate. The sorbent cartridge may include any suitable amount of one or more sorbent materials. In certain instances, the term "sorbent cartridge" can refer to a cartridge which includes one or more sorbent materials in addition to one or more other materials capable of removing waste products from dialysate. "Sorbent cartridge" can include configurations where at least some materials in the cartridge do not act by mechanisms of adsorption or absorption.

The terms "stopping" or to "stop" a dialysis session refer to ceasing treatment of a patient by dialysis.

The term "store" refers to saving a digital representation of a parameter or variable in memory.

A "subcutaneous sensor" is a sensor located underneath a portion of the skin of a patient.

A "surface sensor" is a sensor in contact with an external portion of the body of a patient.

The term "target potassium level" refers to a desired potassium concentration of a patient. The desired potassium concentration of a patient can be a desired potassium concentration of a patient after dialysis, or at any point during a dialysis session. For example, the "target potassium level" can refer to a desired patient potassium concentration after treatment. Alternatively, the "target potassium level' can refer to a desired potassium concentration of a patient at a specified time during a dialysis session.

The term "user selected response" refers to a change in dialysis treatment based on a monitored parameter, wherein the change is predetermined by a user.

### Dialysis Device

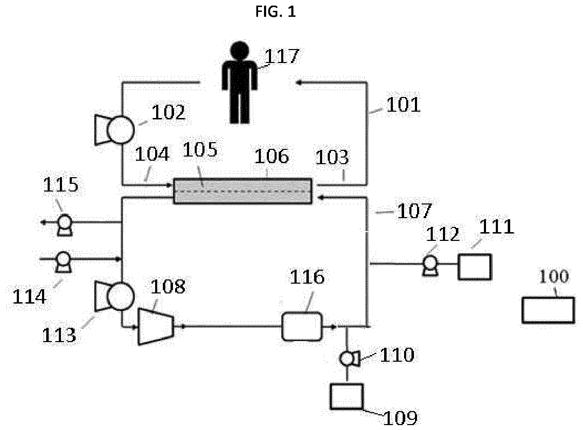

FIG. 1 shows a sorbent based dialysis system including a dialysis apparatus or system including extracorporeal flow path **101,** a dialysate flow path **107,** and a dialyzer **106** containing a membrane **105.** Blood from a patient **117** enters the dialyzer **106** through a blood inlet line **104** and exits through a blood outlet line **103** with a blood pump **102.** The dialysate flow path 107 can be a controlled compliant flow path. The terms controlled compliance and controlled compliant refer to the ability to actively control the transfer of fluid volume into or out of a compartment, flow path or circuit. The variable volume of fluid in a dialysate circuit controlled or compliant flow path can expand and/or contract via the control of one or more pumps in conjunction with one or more reservoirs. The volume of fluid in the system is generally constant (unless additional fluids are added to a reservoir from outside of the system) once the system is in operation if the patient fluid volume(s), flow paths, and reservoirs are considered part of the total volume of the system (each individual volume may sometimes be referred to as a fluid compartment).

As shown in FIG. 1, dialysate travels from the dialyzer **106** through the dialysate flow path **107** and a dialysate regeneration module **108.** The dialysate regeneration module **108** can be any component or set of components capable of removing toxins and waste products from the dialysate, allowing the dialysate to be reused. In certain embodiments, the dialysate regeneration module **108** can be a sorbent based filtration system that removes at least a portion of potassium from the dialysate. The sorbent based filtration system can remove solutes from the dialysate through adsorption by a sorbent material. The sorbent based filtration system can include one or more sorbent cartridges containing one or more sorbent materials, or any other components that can adsorb solutes from the dialysate. The sorbent based filtration system can contain multiple materials selected from the group: 1) a urease-containing material, where urease catalyzes the conversion of urea to ammonium ions and carbon dioxide; 2) a zirconium phosphate (ZrP) material with the capacity to act as a cation exchanger by absorbing a large quantity of ammonium ions, potassium ions, calcium ions and magnesium ions for sodium and hydrogen ions; 3) a hydrous zirconium oxide material (ZrO), which acts as an anion exchanger, such as by exchanging phosphate for acetate; 4) an activated carbon material with a surface area for adsorption of a wide range of impurities, including metal ions and uremic toxins, such as uric acid, creatinine, and p2-microglobin; and 5) other ion-exchange materials for removing cations and anions Ion-exchange materials can include weak and strong acid cation exchange resins, weak and strong basic anion exchange resins, chelating ion exchange resins, or other ion exchange resins known to those skilled in the art. The materials present in the sorbent based filtration system may be arranged in layers in any order in one or more sorbent cartridges, or can be intermixed. Alternative methods of dialysate regeneration can be used, including an electrodialysis unit, or modules that do not include enzymatic conversion of urea to bicarbonate. The systems and methods can also work with other dialysis modes, including single-pass dialysis systems or combinations of single and multi-pass systems.

A potassium source **109** downstream of the dialysate regeneration module **108** can contain potassium ions for addition to the dialysate. A potassium pump **110** can control the movement of potassium concentrate from the potassium source **109** into the dialysate flow path **107.** The potassium source **109** can contain potassium as a liquid, dry powder, highly concentrated media, or combinations thereof. The potassium pump **110** can control addition of potassium to the dialysate by metering in potassium as a liquid concentrate to the dialysate from the potassium source **109** to achieve a specified dialysate potassium concentration. Additional solute sources, such as infusate source **111** can be included to add additional components into the dialysate flow path **107.** Infusate pump **112** is fluidly connected to the infusate source **111** containing an infusate concentrate in a form as liquid, dry powder, highly concentrated media, or any combination thereof. The infusate source **111** can contain calcium, potassium, and magnesium cation concentrates. The infusate source **111** can also contain acetate, bicarbonate, sodium, and other concentrates and serve to reconstitute the dialysate prior to entering the dialyzer **106.** The purpose of potassium and infusate sources is to rebalance and provide the appropriate electrolytes and buffer to the dialysate. The infusate concentrates can be contained in separate containers, such as separate acetate concentrate sources and cation concentrate sources, and need not all be in the single infusate source **111.** For example, two, three, four, or more infusate sources can be provided. The system can alternatively include a dry potassium cartridge containing a potassium salt (not shown), where a potassium concentrate can be produced by metering a portion of the regenerated dialysate through the dry potassium cartridge, and then be re-infused to the dialysate to add potassium as needed.

Gas can be removed from the regenerated dialysate when passing through at least one degasser **116.** The at least one degasser **116** may be positioned to operate prior to the addition of the potassium and infusate concentrates, but also may be positioned elsewhere in the dialysate flow path **107.** Spent dialysate exiting the dialyzer **106** can pass an ultrafiltration system including an ultrafiltration pump **115** and an ultrafiltration reservoir (not shown). The ultrafiltration system can remove ultrafiltrate obtained across the dialyzer **106** from the patient **117** and remove any other fluid added into the dialysate flow path **107,** such as fluid added from the potassium source **109** or infusate source **111.** As necessary, additional water can be added to the dialysate flow path **107** by water pump **114** in fluid communication with a water source (not shown). Source water can come from tap water or other types of water resources.

The flow path of FIG. 1 can also include a controller **100,** which controls the potassium pump **110** to introduce the potassium concentrate from the potassium source **109** into the dialysate flow path **107.** The controller **100** can be a processor in communication with the potassium pump **110** wherein the controller **100** can communicate wirelessly or by direct electrical connection. The controller **100** can include one or more processors, memory, and input/output interface. The memory can be in communication with the processor and store instructions that when executed perform the methods of the present invention. In particular, the controller **100** can control any pumps or components in the system, and any operation such as priming, cleaning, and therapy. The input/output interface can include an input port to receive the patient and system parameters, information from one or more sensors including conductivity sensors, and any other information that can be entered by a patient or health care professional. The input/output interface can also have an output interface to output a calculated rate of addition of potassium from the potassium source **109.**

In a non-limiting embodiment, the input/output interface can be configured between the controller **100** and potassium pump **110** wherein the controller **100** can be programmed to result in a desired potassium level of the patient **117** based on an indicator of potassium levels in the patient **117** obtained from one or more potassium sensors (not shown in FIG. 1). The desired potassium level of the patient **117** can be set as any potassium level within a normal physiological range, including between 3.5 and 5.0 mmol/L. The controller **100** can set or adjust the dialysate potassium concentration to reduce the likelihood of patient arrhythmias based on the monitored potassium level of the patient **117** in real-time and/or on-demand. Sensors can be used to obtain measurements to appropriately adjust the pH or electrolyte level. Data acquired from the sensors may be transmitted to blood fluid removal devices or devices in communication with blood fluid removal devices for adjusting the concentration of pH buffers or electrolytes such as potassium in dialysate or replacement fluid. The pH and electrolyte concentration of the fluid (dialysate or replacement fluid) may be adjusted in any suitable manner. For example, the potassium concentration in the dialysate can be adjusted based on the obtained sensor measurements.

The real-time feature is a capability of the system to receive and respond to an input signal such that any computation and resulting outcome is effected while the input signal is being received. The adjustments can be made continuously or at specified intervals such that a desired outcome is obtained. For example, a constant potassium gradient across the dialyzer **106** or specified rate of potassium delivery or removal from the patient **117** can be effected in real time by receiving the required input signal, computing the desired potassium levels in a patient **117,** and continuously adjusting a pump rate delivering a potassium concentrate into a dialysate on the dialysis machine to effect such specified gradient or rate. The responsive and dynamic adjustment of the real time system can ensure that any fluctuations in potassium delivery or removal can be rapidly addressed during dialysis therapy. Potassium monitoring may occur during a dialysis session, and the concentration or composition of buffer or electrolytes may be adjusted based on monitored data acquired during the dialysis session. By monitoring pH or electrolytes, an appropriate initial dialysate or replacement fluid may be used, or the fluid may be adjusted during a session, to enhance patient safety. In one non-limiting example, the real-time adjustment of the potassium dialysate concentration can be adjusted based on an electrical signal received and computed from an ECG.

The on-demand feature is a capability of the system to respond and provide a specified outcome based on an instruction whether based on inputs or instructions from the controller **100** or an operator/user (not shown). For example, a user or operator can input a desired potassium gradient across the dialyzer **106** or specified rate of potassium delivery or removal from the patient **117.** The desired gradient and rate can be effected based on the instruction to obtain the specified potassium levels in a patient **117.** The controller **100** can then control the potassium pump **110** to adjust the pump rate to deliver a potassium concentrate into a dialysate to result in the specified gradient or rate. The on-demand system can ensure that any fluctuations in potassium delivery or removal can be quickly addressed during dialysis therapy in a clinical setting based on monitored data of potassium levels in a patient **117.**

One or more potassium sensors (not shown) can be included at various points in the dialysate flow path **107** to monitor potassium levels. A potassium sensor downstream of the potassium source **109** can monitor a dialysate potassium concentration and ensure that the actual dialysate potassium concentration is the same as the dialysate potassium concentration set by the controller **100.** A potassium sensor upstream of the dialysate regeneration module **108** can determine the potassium concentration of spent dialysate exiting the dialyzer **106.** A potassium sensor between the dialysate regeneration module **108** and the potassium source **109** can determine the dialysate potassium concentration prior to addition of a potassium concentrate to provide data to the controller **100.** The controller **100** can then calculate the amount of potassium concentrate to be added to the dialysate and instruct the potassium pump **110** to pump a desired amount of potassium into the dialysate. Potassium sensors both downstream of the potassium source **109** and in the extracorporeal flow path **101** can determine the potassium gradient across the dialyzer **106.** In the extracorporeal flow path **101,** a sensor upstream of the dialyzer **106** can provide a direct measurement of patient potassium level. A sensor downstream of dialyzer **106** can, when combined with an upstream sensor, determine how much potassium is removed from the patient **117.**

Sensors are used to monitor pH and electrolytes in the patient **117.** The sensors can monitor pH, electrolytes, or indicators thereof, in the patient's blood. The sensors can also monitor pH or electrolytes, or indicators thereof, in tissue or fluid other than the blood. Other tissues or fluids can tend to equilibrate with blood. As such, changes in pH or electrolyte levels in tissue or fluid other than blood may be indicative of changes of pH or electrolytes in blood. There can be delay in the time of changes in pH or electrolytes in tissue or fluid other than blood relative to actual changes in the blood. Such differences can be computationally accounted for using known techniques. In addition to absolute values of monitored indicators, trends showing downward or upward changes in pH or electrolytes in tissue or fluid other than blood may be correlated to changes in blood pH or blood electrolytes. Sensor can be positioned to detect pH or electrolytes in, for example, a peritoneal cavity or a cerebrospinal fluid compartment of the patient. One non-limiting example of a sensor for an implantable medical device including a potassium sensor is provided in US2007079248. The potassium sensor is a potassium sensitive electrode on a medical electrical lead.

Although shown as a multi-pass sorbent based system in FIG. 1, potassium control can be used with any type of dialysis system such as hemodialysis, hemodiafiltration, and peritoneal dialysis. The dialysis systems can also include closed loop potassium control systems. For peritoneal dialysis, the controller **100** can determine a desired potassium concentration of the peritoneal dialysate and instruct the potassium pump **110** to pump a desired amount of potassium into the peritoneal dialysate prior to introduction to the patient **117.** For use with a single-pass dialysis system, the potassium concentrate can be added directly to source water in a dialysate flow path **107.** Additional concentrates, including sodium, magnesium, calcium, and bicarbonate ions can be added to the source water from one or more additional concentrate sources including a sodium source, a magnesium source, a calcium source, a bicarbonate source, or combinations thereof.

The potassium sensors of the system can be any type of sensor capable of determining the potassium level of the patient **117.** The potassium sensor can be placed anywhere in the patient **117** or system, including on any one of a dialysis machine, external medical device, or point of care component. Alternatively, the potassium sensor can be an implantable sensor, external sensor, subcutaneous sensor, surface sensor, and combinations thereof. Potassium selective electrodes are known in the art and can be used in either an implanted medical device or in the extracorporeal flow path **101** of the dialysis system to provide real-time monitoring of patient potassium levels during treatment. The potassium sensors can be a subcutaneous sensor or a surface sensor in contact with the patient **117.** A controller **100** can be programmed to receive the potassium level of the patient **117** from the sensor through wireless or wired communication. Any suitable ion selective electrode can be configured to detect K+ ions. Electrodes, and components of sensors employing such electrodes, are known in the art and may be employed, or modified to be employed, for use in monitoring as described herein. Additionally, one or more sensors may be employed to detect the potassium level of the patient **117** directly as a potassium concentration in serum or other representative indicator of potassium level in a patient **117.** In some embodiments, a sensor may have more than one electrode, even if leadless, that may monitor potassium ion concentration.

The sensors of the invention can detect patients having a potassium concentration at an elevated or lowered level. More than one sensor can be used for confirmation and/or redundancy to improve reliability and accuracy. The sensors can be configured to accurately detect different ranges of concentrations of the potassium ion or potassium level in the patient **117.** One or more sensors can be present in a single system to provide for convenient data collection and circuitry. Further, plural sensors can be used, if needed or desired, to share the same data processing and memory storage components. The sensor for detecting patient potassium levels can be positioned at any suitable location for purposes of monitoring potassium levels or indicators of potassium level in the patient **117.** For example, the sensor may be implanted in the patient **117,** located external to the patient **117** upstream in a blood fluid removal device, located external to the patient **117** and downstream of the blood fluid removal device, or the like.

ECG signals have been shown to correlate to the potassium level of the patient **117.** In particular, the T-wave right slope, T-wave amplitude, T-wave center of gravity, and the ratio of the T-wave amplitude to the R-wave amplitude have been shown to correlate significantly with potassium, allowing small changes in the potassium level of the patient to be detected. The shape of the ECG signal can be analyzed by the controller **100** to determine the potassium level of the patient **117** in real-time. A global algorithm can be used to determine the potassium level of any patient, or for more accurate results a personalized ECG algorithm can be used. The patient ECG signals can be measured prior to a dialysis session and correlated to patient potassium levels obtained from blood analysis or any other method to create a personalized ECG algorithm. ECG signals include a TP elevation, P amplitude, P wave area, PR interval, PR segment, QRS complex, QRS area, RT duration, R amplitude, ST segment, QT interval, T left slope, T wave area, T center of gravity, T amplitude, T right slope. Of these, five ECG features that can be used for analyzing the potassium level in a patient include: (i) a right slope of the T wave (T right slope), (ii) an amplitude of the T wave (T amplitude), (iii) a center of gravity (COG) of the T wave (T COG), (iv) a ratio of the amplitude of the T wave to amplitude of the R wave (T/R amplitude), and (v) a center of gravity of the last 25% of the area under the T wave curve (T4 COG) (Noninvasive potassium determination using a mathematically processed ECG: Proof of concept for a novel "blood-less, blood test," Dillon et al., J. Electrocadiology 48 (2015) 12-18). The T right slope can be calculated as the mean first derivative of the T wave from the beginning to the end of the TpTe interval. The center of gravity can be set as the mean amplitude (mV)/mean duration (ms). One of ordinary skill can use automatic algorithms to extract the noted features. Additionally, other appropriate features, algorithms, and techniques based on the ECG known to those of ordinary skill in the art can be obtained from a patient **117** using the described sensors. An ECG or other implanted sensor may provide a more accurate potassium level of the physiologic potassium level. As a patient **117** is dialyzed, the blood may show a drop in potassium, but the tissues and the heart may still have high potassium levels until the tissue potassium diffuses into the blood and is pulled out by the dialyzer **106.**

One suitable implantable sensor device that is configured to monitor a patient's ECG signals is a Medtronic, Inc.'s Reveal® series insertable cardiac monitor described above. Other suitable ECG monitoring devices can be used and includes suitably equipped pacemakers or defibrillators already implanted in the patient **117.** Monitored cardiac signals from such a device may be transmitted to a blood fluid removal device or intermediate device for use in the blood fluid removal session having a capability to dynamically adjust the potassium concentration in real-time based on the obtained monitoring. The systems can also optionally set other dialysis prescription variables such as rate and time for the blood fluid removal session based on the adjustments to the dialysate potassium concentration. Blood pressure monitors, which may be external or implantable (such as Medtronic Inc.'s active leadless pressure sensor (ALPS), which generally takes the form of a stent to anchor the device within a vessel, may be employed. The device may be placed in any suitable blood vessel location, such as in a femoral artery or pulmonary artery. A wearable sensor system, such as a Holter sensor system, may be used to monitor ECG activity of the patient **117.**

The ECG and EMG sensors and monitoring systems can also be configured to monitor one or more of heart rate, heart rhythm or a variable thereof, or blood pressure, or any other parameter indicative of potassium levels. Examples of variables of heart rhythm that may be measured are heart rate variability (HRV), heart rate turbulence (HRT), T-wave alternans (TWA), P-wave dispersion, T-wave dispersion, Q-T interval, ventricular premature depolarization (VPD), and any other identified features of the ECG such as slope, center of gravity, amplitudes, and ratios of the waves. The ECG or EGM sensor can be implanted in the patient **117,** either as part of a cardiac management device or as a standalone sensor. A controller **100** can be programmed to receive the ECG or EGM data, and to determine the potassium level of the patient **117.** Alternatively, the ECG or EGM sensor can be a subcutaneous sensor, a surface sensor, or an external sensor located on an external medical device or other point of care component and need not be implanted.

Additionally, potassium concentrations and indicators of potassium concentration indicators parameters can include any patient metric automatically sensed or measured or obtained by implantable or non-implanted sensors, or detection component. Monitoring may alternatively or additionally include receiving patient or physician feedback regarding the patient's state. For example, the patient **117** may indicate a point in time when cramping begins, which often happens when too much fluid is removed. The blood fluid monitoring device may include an input, such as a keyboard or touch screen display for entering such data. Alternatively, a separate device such as a patient programmer, laptop computer, tablet computer, personal data assistance, smart phone or the like may be used to input the data; or the like.

Regardless of whether the sensor or sensor system employed, or components thereof, is implantable, wearable, part of a larger stand-alone device, or part of a blood fluid monitoring device, the sensor may monitor any suitable parameter indicative of potassium levels in the patient **117** can be used in the present invention. Also, multiple sensors may be connected via telemetry, body bus, or the like. The connected sensors may be of the same or different type (e.g., pH or impedance). Such connected sensors may be placed (e.g., internal or external) for purposes of monitoring at various locations of the patient's body.

### Potassium Control

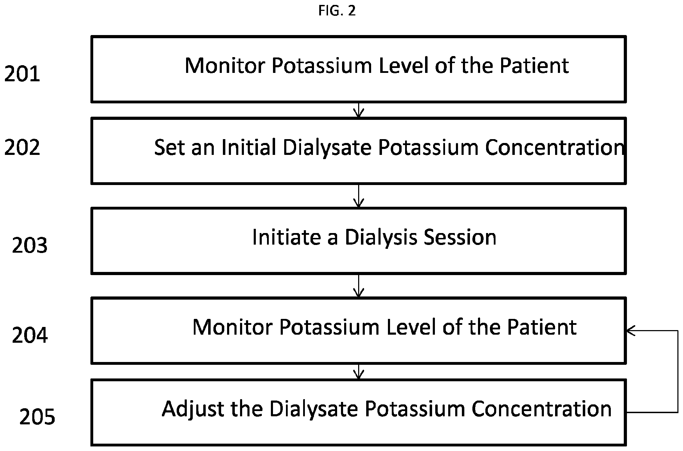

FIG. 2 illustrates a control algorithm for controlling the dialysate potassium concentration based on a monitored potassium level of the patient. Prior to a dialysis session the potassium level of the patient can be monitored in step **201.** The initial potassium level of the patient can be used to set the initial dialysate potassium concentration in step **202.** In some embodiments, an initial buffer or electrolyte concentration or composition of a fluid for use in the blood fluid removal process may be selected based on data acquired from the sensors. Any suitable device or system for removing fluid, or fluid and contaminants, from blood may be used in accordance with the teachings presented herein. The devices, or components thereof, may be traditional large counsel-type, wearable, or implantable. The methods, systems and devices described herein may be used, in some embodiments, to set the initial electrolyte concentration and pH (buffer components and concentration) based on monitoring that occurs before a blood fluid removal session starts. In some embodiments, the monitoring is chronic; e.g., monitoring is performed intermittently, periodically or continuously over the course of days, weeks, months or years. In an attempt to minimize interference with the patient's lifestyle, the monitoring system, or components thereof, may be implantable or wearable. The initial dialysate potassium concentration can be set to a value lower than the potassium level of the patient to remove potassium from the patient, but at a high enough level to avoid drastic changes in patient potassium levels, which can lead to arrhythmia. A dialysis session can be initiated in step **203** using the initial dialysate potassium concentration set in step **202.** During the dialysis session, the potassium level of the patient can be monitored in step **204.** The in-session monitoring can be continuous or at set intervals of time, including every minute, every 5 minutes, every 10 minutes, or any other interval. Based on the monitored potassium level of the patient, the dialysate potassium concentration can be adjusted in step **205** by adjusting the rate at which potassium concentrate is added to a dialysate flow path from a potassium source. As the session continues, the system can monitor the potassium level of the patient continuously and/or repeatedly, and adjust the potassium level accordingly, providing real-time control over the potassium gradient between the blood of the patient and the dialysate. By keeping the potassium gradient below a predetermined threshold, the system can reduce the likelihood of arrhythmia due to changes in patient potassium levels. The predetermined threshold can be based on the patient's dialysis prescription and is set by a physician specific to each patient, or can be set based on data from larger populations. The system can adjust the dialysate potassium concentration in step **205** to maintain a constant or near constant potassium gradient. Alternatively, the system can adjust the dialysate potassium concentration in step **205** to keep the potassium gradient within some predetermined range. The predetermined range can be any range at which adequate potassium removal occurs while reducing the probability of patient arrhythmia. The predetermined range can be set by a physician specific to the needs of each patient, or set based on data from larger populations.

The system can also receive a target potassium level of the patient for the dialysis session. The target potassium level of the patient can be used in step **205** to set the dialysate potassium concentration at a level that will result in the target potassium level of the patient after dialysis. Ione goal of hemodialysis, ultrafiltration, and the like is to ensure that the patient's blood pH and electrolyte concentrations are within acceptable ranges. Typical ranges of potassium concentration that are desired during or following a dialysis session are between 3.5 and 5.0 mmol/L. However, it will be understood that the target for a particular patient may be different from the values presented. Once the target potassium level of the patient is achieved, the system can adjust the potassium concentration of the dialysate to prevent the potassium level of the patient from decreasing beyond the target. Dialysis can continue without the risk of the patient's potassium level dropping below the target potassium level of the patient.

The dialysate potassium concentration control can be a closed loop potassium control system. As used herein, the terms "closed loop potassium control" and "open loop potassium control" refer to potassium control and do not refer to single pass or regenerative systems. A closed loop potassium control system uses one or more potassium sensors to control the level of potassium in the dialysate and thus control the amount of potassium removed from the patient or control the patient's final potassium level. In a closed loop potassium control system, the controller can adjust the dialysate potassium concentration automatically in response to the changing patient potassium level. An open loop potassium control system uses a set potassium level in the dialysate without regard to the patient potassium level. The system can alert the user if the potassium gradient is above or below a predetermined threshold, and the user can manually input a new dialysate potassium concentration. With a closed loop potassium control system, the system can provide alerts to the user indicating that the potassium gradient is above or below a predetermined threshold, that the patient potassium level has dropped below a predetermined threshold, or that the patient potassium level is changing at too fast a rate. The system can automatically stop treatment if the patient potassium level drops below a predetermined threshold.

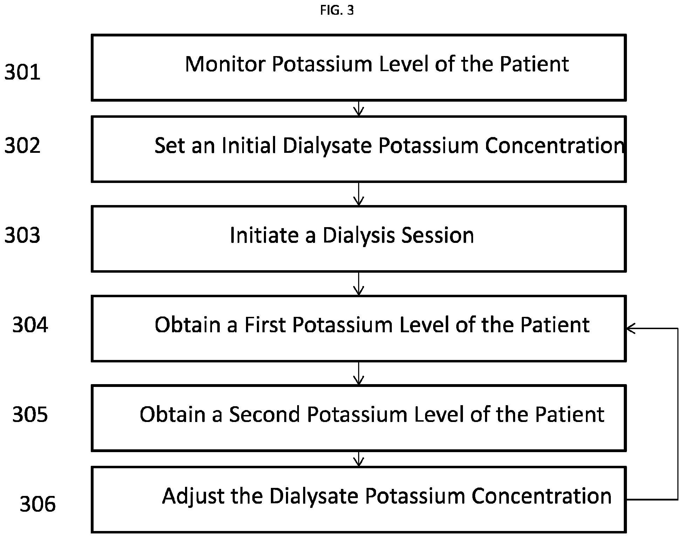

FIG 3 illustrates an alternative control algorithm based on a rate of change of the patient potassium level. In step **301** the patient potassium level can be monitored prior to starting the dialysis session. The initial potassium level of the patient can be used to set the initial dialysate potassium concentration in step **302.** The initial dialysate potassium concentration can be set to a value lower than the potassium level of the patient to remove potassium from the patient, but at a high enough level to avoid drastic changes in patient potassium levels, which can lead to arrhythmia. A dialysis session can be initiated in step **303** using the initial dialysate potassium concentration set in step **302.** During the dialysis session, the potassium level of the patient can be monitored in step **304.** A second potassium level of the patient at a later point in time can be obtained in step **305** to provide a rate of change of the patient potassium level. Based on the rate of change of the patient potassium level, the dialysate potassium concentration can be adjusted in step **306.** If the system detects a rate of change in the patient potassium level above a predetermined threshold, the potassium concentration of the dialysate can be increased, slowing the movement of potassium ions from the patient's blood to the dialysate. In certain circumstances, the potassium concentration of the dialysate can be increased to a value greater than the potassium level of the patient to reverse the movement of potassium, providing additional potassium ions to the patient. The algorithm can repeat, starting with step **304,** and the rate of change in the patient potassium level can continuously be monitored and updated in real-time. The system can stop treatment or alert a user if the rate of change in the patient potassium level crosses a predetermined threshold.

The predetermined range for the change in the potassium level of the patient can be set to any range in which the probability of arrhythmia is reduced. The range can be... The lower end of the range can be set to minimize treatment time and ensure adequate potassium removal from the patient. Although a smaller rate of change in potassium level may further reduce the probability of arrhythmia, too small of a rate of change would require a longer treatment time to remove the necessary amount of potassium. The lower end of the predetermined range can be set taking into account the patient's starting potassium level, as well as the total amount of potassium that should be removed from the patient. The upper end of the predetermined range can be set to any value below the point at which a significant increase in probability of arrhythmia occurs.

The potassium control algorithms illustrated in FIG.'s 2 and 3 can be combined into a single algorithm. The system can adjust the potassium concentration of the dialysate to maintain a concentration gradient within a predetermined range. At the same time, the system can also monitor the rate of change of the potassium level of the patient, and adjust the dialysate concentration to keep the rate of change within a predetermined range.

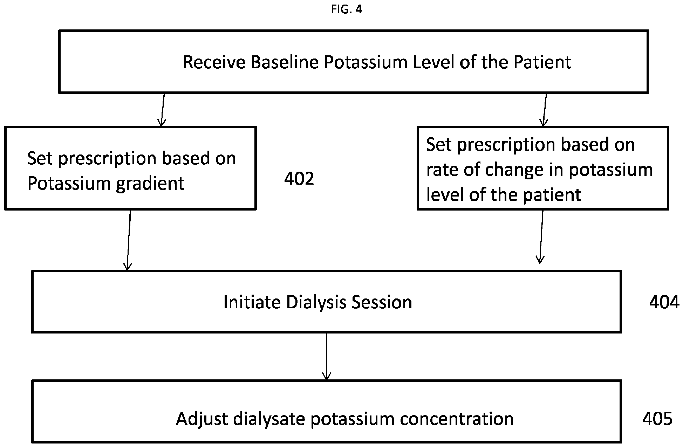

FIG. 4 illustrates a control algorithm for setting a prescription for changing the potassium level of a patient. In step **401,** the system can receive a baseline potassium level of the patient. The baseline potassium level of the patient is the potassium level of the patient prior to dialysis. Using the baseline potassium level of the patient, the system can set the prescription to change the potassium level of the patient. In step **402,** the prescription can include a defined potassium gradient across a dialyzer. Based on the baseline potassium level of the patient the defined potassium gradient across the dialyzer can be set to a level that will result in a decreased likelihood of arrhythmia. The defined potassium gradient across the dialyzer can be set to a predetermined threshold that will allow the prescribed amount of potassium to be removed from the patient without increasing the likelihood of arrhythmia by removing potassium too quickly. In step **403,** the prescription can include a defined rate of change in the patient potassium level. The defined rate of change in patient potassium level can be set at a predetermined threshold that will likewise remove the prescribed amount of potassium without increasing the likelihood of arrhythmia.

The system can also set the prescription for changing the potassium level of the patient using both methods illustrated in steps **402** and **403.** For example, the prescription can include a defined potassium gradient across the dialyzer in step **402** and a defined rate of change in patient potassium level in step **403.** In step **404,** the system can initiate the dialysis session, and adjust the dialysate potassium concentration as necessary in step **405** to maintain the defined potassium gradient across the dialyzer and/or the defined rate of change in patient potassium level throughout the dialysis session. The system can then reset the defined rate of change in patient potassium level in step **403** or the defined potassium gradient in step **402** and adjust the dialysate potassium level again as needed in step **405,** as illustrated by the loop back from step **405** to each of step **402** and **403.**

In addition to adjusting the potassium concentration of the dialysate, other solutes in the dialysate can be adjusted based on the monitored potassium level of the patient. Dialysate glucose and/or bicarbonate can affect the potassium level of the patient. Adjusting the dialysate glucose and/or dialysate bicarbonate concentration by adding a glucose concentrate from a glucose source, or a bicarbonate concentrate from a bicarbonate source, can lower the risk of patient arrhythmia while removing potassium from the patient, which beneficially allows more potassium to be removed from the patient without an increased risk of arrhythmia.

The dialysis system can adjust the dialysate potassium concentration in accordance with predetermined rules, such as to keep the concentration gradient between the patient's blood and the dialysate between predetermined thresholds. Alternatively, a user may include a user selected response to the potassium level of the patient. Allowing a user to program the system response to the monitored potassium level of the patient provides a personalized potassium control system, specific to each patient.

To facilitate a user selected response to the potassium level of the patient, the processor or controller can receive additional patient or system parameters, including the sodium level of the patient, the blood flow rate for the dialysis session, the dialysate flow rate for the dialysis session, the patient blood pressure, and the patient pulse rate. The processor can store the additional parameters, along with the potassium level of the patient. The system can include an output, which can output a plot of the potassium level of the patient throughout a dialysis session with one or more additional parameters. The plot of the potassium level of the patient and additional parameters can inform the user of the patient's particular response to changing potassium levels. Concentration can also be adjusted by, for example, adjusting the rate at which dialysate or blood is passed over a dialysis membrane. The rate of transfer between blood and dialysate of electrolytes, etc. across the membrane will be dependent on the flow rate of the blood and the dialysate. Accordingly, the rate of flow of blood or dialysate flow may be altered to achieve similar effects to adjusting the concentration of electrolytes in dialysate. As a non-limiting example, if a particular patient maintains a relatively steady heart rate while the patient potassium levels decrease, the user may select a more aggressive response to the monitored potassium level of the patient in the same or future dialysis session, increasing the rate of change in patient potassium level for the particular patient and potentially reducing the required treatment time.

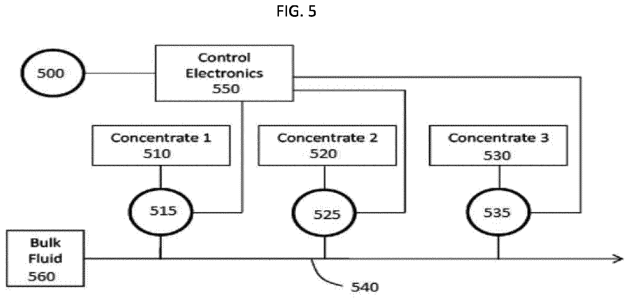

FIG. 5 illustrates non-limiting representative components of an example of a closed-loop control system for adjusting pH and electrolyte concentrations of fluid. Data from sensor **500** is presented to control electronics **550,** which are programmed or designed to control flow control elements **515, 525, 535,** such as valves. The electronically controllable flow control elements **515, 525, 535** are in fluid communication with supplies of electrolyte or buffer concentrates **510, 520, 530** including potassium, and with fluid line **540,** which may be a catheter for carrying fresh dialysate or a catheter for carrying replacement fluid. The electronically controllable flow control elements **515, 525, 535,** via control electronics **550,** control the rate at which the concentrates **510, 520, 530** flow into the fluid line **540.** The
concentrates **510, 520, 530** are added to bulk fluid **560** to adjust the concentration of electrolytes such as potassium or the pH of the bulk fluid (and thus the blood).

One skilled in the art will understand that various combinations and/or modifications and variations can be made in the described systems and methods depending upon the specific needs for operation. Moreover features illustrated or described as being part of an aspect of the invention may be used in the aspect of the invention, either alone or in combination.

The invention may be described by reference to the following numbered paragraphs:-
1. A method, comprising the steps of:
   a) initiating a dialysis session for a patient;
   b) monitoring a potassium level using a sensor;
   c) adjusting a dialysate potassium concentration during the dialysis session based on the monitored potassium level; wherein the dialysate potassium concentration is adjusted to result in a potassium level of the patient within 3.5 - 5.0 mmol/L.
2. The method of paragraph 1, wherein the dialysis session is initiated on a closed loop potassium control system.
3. The method of paragraph 1, wherein the step of monitoring a potassium level comprises monitoring a potassium level of the patient.
4. The method of paragraph 1, wherein the step of monitoring a potassium level comprises monitoring a dialysate potassium concentration.
5. The method of paragraph 1, wherein the step of monitoring a potassium level comprises monitoring a potassium gradient across a dialyzer.
6. The method of paragraph 1, further comprising the step of monitoring a potassium level of the patient prior to initiating the dialysis session, and setting an initial dialysate potassium concentration based on the monitored potassium level of the patient.
7. The method of paragraph 3, wherein the step of monitoring the potassium level of the patient uses an implanted sensor.
8. The method of paragraph 3, wherein the sensor is an ECG or an EGM sensor.
9. The method of paragraph 7, wherein the implanted sensor is a potassium sensor.
10. The method of paragraph 3, wherein the step of monitoring a potassium level of the patient is performed by a sensor on any one of a dialysis machine, external medical device, or point of care component.
11. The method of paragraph 3, wherein the step of monitoring a potassium level of the patient is performed by any one of an implantable sensor, external sensor, subcutaneous sensor, surface sensor, and combinations thereof.
12. The method of paragraph 1, wherein adjusting the dialysate potassium concentration comprises adjusting a rate of addition of a potassium concentrate to a dialysate.
13. The method of paragraph 3, wherein monitoring the potassium level of the patient and adjusting the dialysate potassium concentration are repeated throughout the dialysis session.
14. The method of paragraph 1, wherein the method is performed by a dialysis system.
15. The method of paragraph 1, wherein the dialysate potassium concentration is adjusted to maintain a rate of change of the potassium level of the patient below a predetermined threshold.
16. The method of paragraph 1, wherein the step of adjusting the dialysate potassium concentration comprises adjusting a rate of addition of a potassium concentrate from a potassium source into a dialysate flow path.
17. The method of paragraph 1, wherein the dialysate potassium concentration is adjusted to maintain a constant potassium gradient across a dialyzer.
18. The method of paragraph 1, further comprising adjusting a concentration of at least one other solute in a dialysate based on the monitored potassium level of the patient.
19. The method of paragraph 18, wherein the at least one other solute is either glucose or bicarbonate.
20. The method of paragraph 1, further comprising monitoring a dialysate potassium concentration with a potassium sensor in a dialysate flow path downstream of a dialyzer.

## Claims

1. A method, comprising the steps of:
c) initiating a dialysis session for a patient;
d) monitoring a potassium level using a sensor;
e) adjusting a dialysate potassium concentration during the dialysis session based on the monitored potassium level; wherein the dialysate potassium concentration is adjusted to result in a potassium level of the patient within 3.5 - 5.0 mmol/L.

2. The method of claim 1, wherein the dialysis session is initiated on a closed loop potassium control system.

3. The method of claim 1, wherein the step of monitoring a potassium level comprises monitoring a potassium level of the patient.

4. The method of claim 1, wherein the step of monitoring a potassium level comprises monitoring a dialysate potassium concentration.

5. The method of claim 1, wherein the step of monitoring a potassium level comprises monitoring a potassium gradient across a dialyzer.

6. The method of claim 1, further comprising the step of monitoring a potassium level of the patient prior to initiating the dialysis session, and setting an initial dialysate potassium concentration based on the monitored potassium level of the patient.

7. The method of claim 3, wherein the step of monitoring the potassium level of the patient uses an implanted sensor.

8. The method of claim 3, wherein the sensor is an ECG or an EGM sensor.

9. The method of claim 7, wherein the implanted sensor is a potassium sensor.

10. The method of claim 3, wherein the step of monitoring a potassium level of the patient is performed by a sensor on any one of a dialysis machine, external medical device, or point of care component.

11. The method of claim 3, wherein the step of monitoring a potassium level of the patient is performed by any one of an implantable sensor, external sensor, subcutaneous sensor, surface sensor, and combinations thereof.

12. The method of claim 1, wherein adjusting the dialysate potassium concentration comprises adjusting a rate of addition of a potassium concentrate to a dialysate.

13. The method of claim 3, wherein monitoring the potassium level of the patient and adjusting the dialysate potassium concentration are repeated throughout the dialysis session.

14. The method of claim 1, wherein the method is performed by a dialysis system.

15. The method of claim 1, wherein the dialysate potassium concentration is adjusted to maintain a rate of change of the potassium level of the patient below a predetermined threshold.

16. The method of claim 1, wherein the step of adjusting the dialysate potassium concentration comprises adjusting a rate of addition of a potassium concentrate from a potassium source into a dialysate flow path.

17. The method of claim 1, wherein the dialysate potassium concentration is adjusted to maintain a constant potassium gradient across a dialyzer.

18. The method of claim 1, further comprising adjusting a concentration of at least one other solute in a dialysate based on the monitored potassium level of the patient.

19. The method of claim 18, wherein the at least one other solute is either glucose or bicarbonate.

20. The method of claim 1, further comprising monitoring a dialysate potassium concentration with a potassium sensor in a dialysate flow path downstream of a dialyzer.
